# EUROPEAN PATENT SPECIFICATION

(11) **EP 3 029 715 B1**
(45) Date of publication and mention of the grant of the patent: **16.01.2019**
(21) Application number: 14832130.0
(22) Date of filing: 31.07.2014
(51) Int. Cl.: G01N 1/40, G01N 33/36, G01N 27/62

(54) **METHOD USED FOR RAPID TESTING OF HIGHLY VOLATILE SUBSTANCES OF VERY HIGH CONCERN IN TEXTILES**
VERFAHREN ZUR SCHNELLEN PRÜFUNG VON HOCHFLÜCHTIGEN, SEHR BESORGNISERREGENDEN STOFFEN IN TEXTILIEN
PROCÉDÉ UTILISÉ POUR UNE ANALYSE RAPIDE DE SUBSTANCES HAUTEMENT VOLATILES CANDIDATES À AUTORISATION

(30) Priority: 01.08.2013 CN 201310331950
(43) Date of publication of application: 08.06.2016
(73) Proprietor: Nuctech Company Limited, TongFang Building, Shuangqinglu, Haidian District Beijing 100084 (CN); Changzhou Entry-Exit Inspection and Quarantine, Jiangsu 213003 (CN)
(72) Inventor: ZHANG, Zhongxia, Beijing 100084 (CN); WU, Zeying, Beijing 100084 (CN); ZHANG, Yangtian, Beijing 100084 (CN); QIU, Yuejin, Beijing 100084 (CN); HE, Wen, Beijing 100084 (CN); XUE, Xin, Beijing 100084 (CN); PENG, Hua, Beijing 100084 (CN); ZHANG, Tong, Beijing 100084 (CN); YU, Haijun, Beijing 100084 (CN)
(74) Representative: Carpmaels & Ransford LLP
(86) International application number: PCT/CN2014/083387
(87) International publication number: WO 2015/014294

(56) References cited:
- WO-A2-2008/067395
- CN-A- 102 297 791
- DE-A1-102008 062 436
- US-A1- 2003 193 338
- US-A1- 2005 051 719
- US-A1- 2006 219 892
- CUADROS-RODRGUEZ L ET AL: "Calibration in chemical measurement processes. II. A methodological approach", TRAC TRENDS IN ANALYTICAL CHEMISTRY, ELSEVIER, AMSTERDAM, NL, vol. 20, no. 11, 1 November 2001 (2001-11-01), pages 620-636, XP004313497, ISSN: 0165-9936, DOI: 10.1016/S0165-9936(01)00111-X
- WANG, JIANFENG ET AL.: 'Detection of malathion and dichlorovos in cherry tomatoes using a hand-held ion mobility spectrometer' CHINESE JOURNAL OF ANALYSIS LABORATORY vol. 4, no. 30, 15 April 2011, pages 30 - 33, XP008182065
- WU, ZEYING ET AL.: 'Fast analysis of substances of very high concern in textiles by ion mobility spectrometry' TEXTILE DYEING AND FINISHING JOURNAL vol. 35, no. 11, 20 November 2013, pages 50 - 53, XP008182087

## Description

This application claims priority of China Patent Application No. 201310331950.8, filed on August 1, 2013, entitled "Method for Rapidly Detecting Volatile Substances of Very High Concern in Textiles", the entire contents of which are incorporated in this present application by reference.

### TECHNICAL FIELD

This invention relates to a method for rapidly detecting volatile substances of very high concern (SVHCs) in textiles using an ion mobility spectrometry technique.

### BACKGROUND ART

The REACH Regulation enacted and being implemented in stages by the European Union, which has become a major export market for textiles and related light industrial products of China, has greatly affected China's textiles export. Since June 1, 2009, REACH Regulation No. 1907/2006 Annex XVII has formally replaced the former EU directives. The new regulation lists 38 kinds of substances of very high concern (SVHCs) which will be gradually limited and finally prohibited to be used in a certain period. According to Article 7(2) in REACH Regulation related to SVHC, if an article contains a certain component which is identified as a substance of very high concern and both conditions are met (the total amount of this component contained in these articles produced or imported is over 1 tonne per year; and the mass fraction of this component in these articles is over 0.1%, i.e., 1000ppm), all producers or importers of this article shall notify the authorities of REACH or EU member states about the detailed data of the substance. However, in 55 recall cases which have been disclosed by the European Union so far, recalls involving Chinese textiles comprise 54.55% resulted from a product detection rate of less than 50% on domestic textile market in China. Therefore, there is an urgent demand for the safety control and detection of the exported textiles.

In the list of these 38 SVHCs, volatile organic substances occupy a considerable proportion, such as phthalates, coal tar fractions, some dye intermediates (2,4-dinitrotoluene, 4,4'-methylenedianiline), and auxiliary raw materials (acrylamide, trichloroethylene), etc. Some mature detection methods such as GC/MS, HPLC, etc have been developed for the detection of these volatile organics. At present, as for the determination of phthalates in textiles, there are national standard GB/T 20388-2006 used in China and BS EN 15777:2009 Textiles - Test method for Phthalates applied in foreign countries, etc. Gas Chromatography coupled with Mass Spectrometry (GC/MS) (GC/MS) is specified in most current international and domestic standards as the analytical method used for the detection of phthalates. The main technical procedure include cutting up a textile sample, extracting the target analytes via are flux extraction with an appropriate solvent, and analyzing the purified and concentrated extract using a GC/MS. Domestic detection standards for 2,4-dinitrotoluene, which is a dye intermediate in textiles, mainly include GB/T 17592-2006, determination of prohibited azo dyes in textiles; and BS EN 14362-2003 Textiles - Methods for the determination of certain aromatic amines derived from azo colorants, etc. The detection methods are relatively similar, and the main technical measure is in that a sample is soaked with a citrate buffer at a certain temperature after being cut up, 2,4-dinitrotoluene in a dye is then obtained by reduction with rongalite, followed by extraction with a suitable organic solvent, and GC/MS analysis or HPLC is performed for qualitative and quantitative detection after purification and concentration.

An accurate qualitative and quantitative analysis of the target analytes in textiles can be achieved by using any of these above-described standards/methods for detecting volatile SVHCs. However, there exist many deficiencies including a complex sample pretreatment step, long detection time, high requirements for the equipment and large solvent consumption. If various detection items are desired to be performed, a high detection cost is required, which exerts great pressure on textile production corporations, thereby leading to an urgent demand to reduce the detection cost and shorten the detection period. The ion mobility spectrometry (IMS), as a trace detection technique originated in the 1960s, has been described in detail in some early patents (US 3699333 and 4777363). Featuring a simple structure, and high detection sensitivity (pg-ng level detection limit), the technique is, particularly suitable for trace detection of some volatile organic compounds. Hence, in the past over 20 years, many domestic and foreign academic institutions and companies have made a large number of investigations on practical applications of the IMS technique, particularly on applications in trace detection of illegal drugs, explosives, chemical warfare agents and biological warfare agents, etc. (RU2288459, WO2004018994, US6477907, CA2043825, US2005278142, KR20020017491, etc), and have achieved considerable development. Meanwhile, its scope of applications has been expanded to various fields including environmental detection (EP1006372, US6969851, etc), industrial process control (WO2004016850, US2002121349, CA2097269, CA2069029, CA2009062, EP0683392, etc), biomedicine (DE10339015, US6365339, USH1563H, US4988628, etc), food monitoring (DE10339014, EP1233267, etc), and the like.

However, there have been few studies on the application of IMS in the field of detection of SVHCs in textiles.

US2003193338A1 relates to an IMS detection system that is designed to sample vapors at a distance from the sampling inlet to avoid contamination of the sampling inlet. US2006219892A1 relates to an IMS-based detection method in which the sample gas flow is mixed with a reference gas and a reaction gas is supplied via a metering unit when complex ion-mobility spectrometer spectra are present, where the measurement signals obtained as to the gas-quantity ratio between the sample gas and the reference gas are adapted to the original concentration and are then compared to previously defined and stored measured values via a signal height comparison or a pattern recognition.

### SUMMARY OF INVENTION

With respect to the deficiencies present in existing detection techniques for volatile SVHCs in textiles described above, this invention provides a method for rapidly detecting volatile SVHCs in textiles, as defined in appended claims.

According to the present invention, the detection of 11 kinds of volatile SVHCs may be achieved within 1-6 minutes, respectively, by means of direct introduction of textile samples or by combining an efficient and convenient solvent extraction pretreatment step. The detection limit is less than 100ppm. This method may greatly shorten the detection periods of these volatile organics and enable the possibility to realize online detection of these substances. The method of this invention may be used as an approach for rapid screening of volatile SVHCs in a textile to greatly shorten its detection period and reduce the detection cost.

### BRIEF DESCRIPTION OF THE DRAWINGS

Fig. 1 is a simplified schematic diagram of the construction and principle of an ion mobility spectrometer.
Fig. 2 is a structural schematic diagram of a textile sample clamper.
Fig. 3 is a schematic diagram of the structure of a sample inlet of an ion mobility spectrometer and direct introduction of a textile sample by using the sample clamper.
Fig. 4 is superimposed ion mobility spectra of 11 SVHC standard samples (100ppm) measured in positive and negative mode, wherein the horizontal axis shows the drift time (ms) and the longitudinal axis shows the intensity of ion current signal (mv).

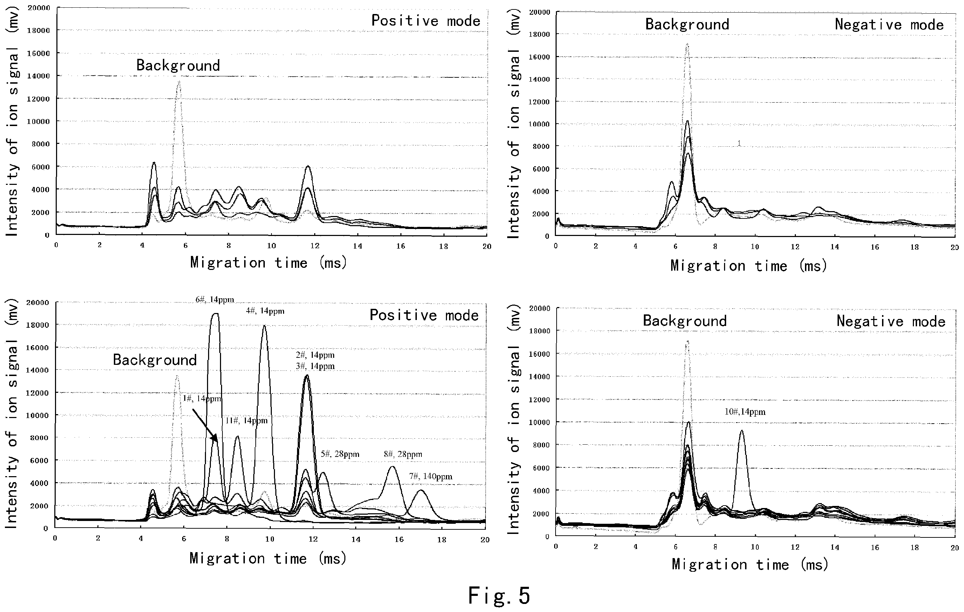

Fig. 5 is ion mobility spectra obtained by an ion mobility spectrometer using the method provided in embodiment 1, wherein the two upper diagrams are the ion mobility spectra detected in both positive and negative mode, obtained by means of direct introduction of blank samples of cotton spandex cloth which are confirmed to be free of 11 volatile SVHCs using a specifically designed clamper; and the two lower diagrams are the superimposed ion mobility spectra detected in both positive and negative mode, obtained by means of direct introduction of spiked cotton spandex cloth samples on which 10 kinds of volatile SVHCs are deposited respectively using a specifically designed clamper, wherein the horizontal axis shows the drift time (ms) and the longitudinal axis shows the intensity of ion current signal (mv).

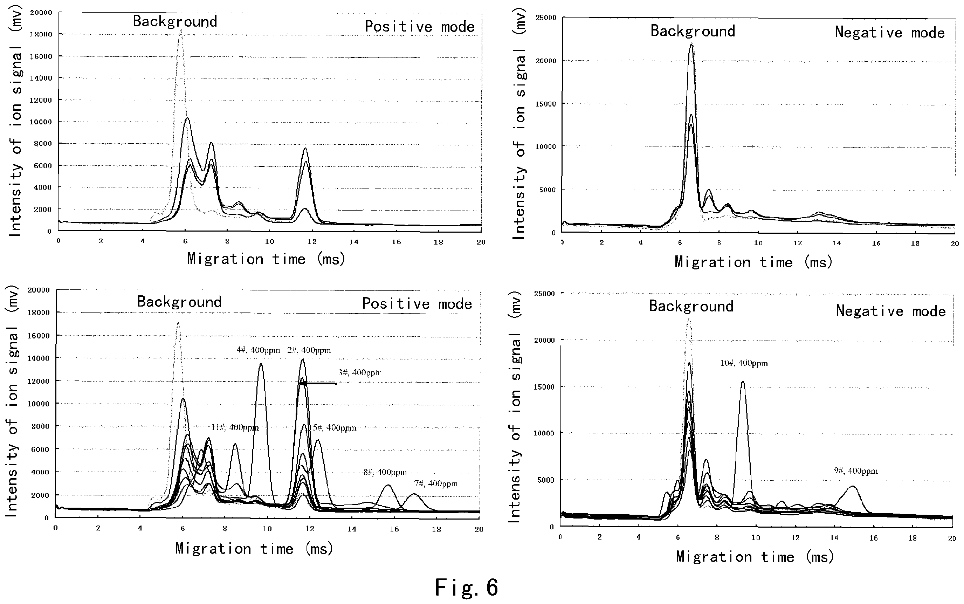

Fig. 6 is ion mobility spectra obtained by an ion mobility spectrometer using the method provided in embodiment 2, wherein the two upper diagrams are the ion mobility spectra detected in both positive and negative mode, obtained by means of performing a simple solvent extraction on a blank sample of wool fabric which is confirmed to be free of 11 volatile SVHCs; and the two lower diagrams are the superimposed ion mobility spectra detected in both positive and negative mode, obtained by means of performing simple solvent extraction operations on spiked wool fabric samples on which 11 kinds of volatile SVHCs are deposited respectively, wherein the horizontal axis shows the drift time (ms) and the longitudinal axis shows the intensity of ion current signal (mv).

### DESCRIPTION OF EMBODIMENTS

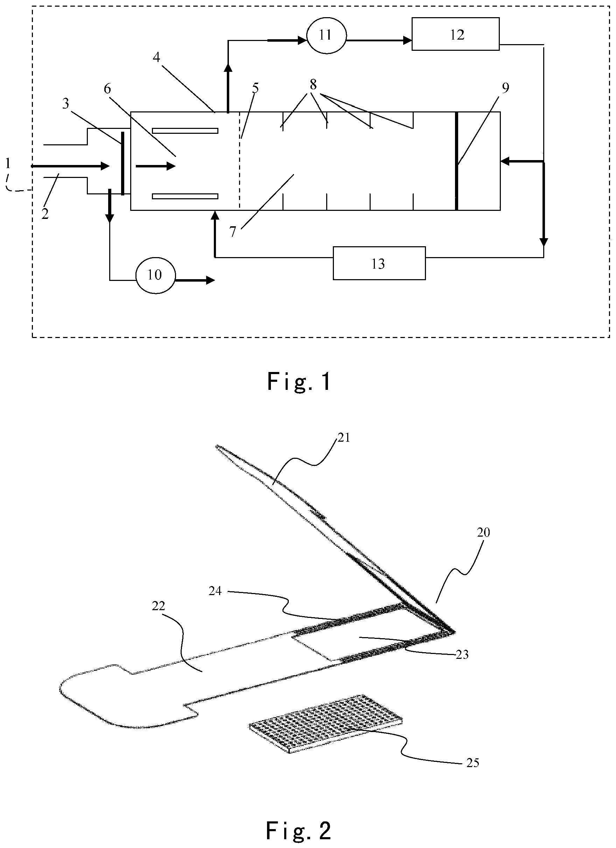

Hereafter, the technical solutions of this invention will be further specifically illustrated by Examples in conjunction with Figs. 1-3. In the specification, the same or similar parts are identified with the same or similar reference numerals. The description of the embodiments of this invention with reference to figures is intended to explain the general inventive concept of this invention and should not be construed to limit the scope of the claims to the disclosed embodiment.

Fig. 1 is a schematic diagram of the construction and principle of an ion mobility spectrometer according to one specific embodiment of this invention. Said ion mobility spectrometer 1 may be a conventional uniform-field device as shown in Fig. 1. There is a sample inlet 2 at one end thereof, which may be used to collect air samples containing the analytes of interesting vapor phase or introduce a sampling substrate carrying particulate samples collected to the instrument. A thermal desorber is provided at the sample inlet end where the particulate samples on the sampling substrate is heated and vaporized. A semipermeable membrane 3 with permselectivity is provided within the sample inlet which separates the interior of the instrument from the sample introduction unit connected to the external environment. Molecules of the gaseous analytes to be tested which are introduced from the sample inlet 2 in different sample introduction modes are entrained to the front side of the semipermeable membrane by air flow provided by a sampling pump 10. The analyte molecules then selectively diffuse through the membrane into an ion drift tube 4 which is a core element in the instrument. The components which cannot permeate the membrane are discharged by the sampling pump. The drift tube 4 is partitioned into two parts, that is, an ionization reaction region 6 and a drift region 7, separated by an ion gate 5 which may be periodically opened. A uniform electric field is provided by applying voltage on annular electrodes 8 disposed in the drift region. In the ionization region of the ion drift tube, the analyte molecules are ionized to form ion clusters. When the ion gate is opened, these ion clusters are injected into the drift region and migrate forward under the influence of the electric field. Since the ion clusters migrate through the drift region with different velocities which are dependent upon their masses, size, charges, etc., they arrive at a detector 9 located at the end of the drift region at different times. The substance species may be identified by comparing the weak pulse current and the arrival time values measured by the detector with the data stored in the standard substance library. The drift gas introduced from the rear end of the instrument flows in the direction opposite to the ion movement and is guided out from an exit near the ion gate in the ionization chamber. Under the propelling action of a circulating pump 11, a part of the gas purified and dried by a filter 12 is used as the drift gas introduced into the rear part of the drift region, while the other part is introduced into the ionization reaction region via a gas path containing a dopant source 13. The detection mode of the instrument is determined by the polarity of the electric field in the drift region 7. When the polarity is positive, negative ions generated in the ionization region 6 are injected into the drift region 7 for separation and detection to obtain a negative-mode ion mobility spectrum. Conversely, when the polarity is negative, positive ions generated in the ionization region 6 are pulsed into the migration region 7 for separation and detection to obtain a positive-mode ion mobility spectrum. Adopting the design mode using either a single drift tube operated with rapidly alternating polarity or dual drift tubes operated with opposite polarities, simultaneous detection of positive and negative ions may be achieved to obtain ion mobility spectra in both positive and negative mode. The method for rapidly detecting volatile SVHCs in a textile according to the Examples of this invention may comprise two parts, that is, the establishment of a standard substance library for volatile SVHCs in a textile r and the detection of textile samples using an ion mobility spectrometer.

In order to detect textiles to be tested, the method for rapidly detecting volatile SVHCs in a textile according to the Examples of this invention comprises: with respect to different types of textiles and volatile SVHCs, performing detection either by means of directly inserting the textile sample to a sample inlet of the spectrometer 2, or in combination with a coefficient and convenient solvent extraction pretreatment step, by depositing the obtained extract onto a sampling substrate and inserting the substrate into the sample inlet of the spectrometer 2; establishing a standard library of various volatile SVHCs based upon the obtained ion mobility spectra of a series of spiked textile samples; and if a peak characteristic of a volatile SVHC is present in the ion mobility spectrum of the textile sample and the peak intensity exceeds or is equal to its corresponding threshold value, determining the textile sample to be positive and generating an alarm; and if in the ion mobility spectrum of the textile sample no peak characteristic of a volatile SVHC is observed or a peak characteristic of said volatile SVHC is present but the peak intensity of this peak is less than said threshold value, determining the textile sample to be negative; and estimating the concentration of the volatile SVHC in the textile sample based on the weight of the textile sample and the intensity of the peak characteristic of the volatile SVHC in the ion mobility spectrum.

As for SVHCs with relatively high volatility and textiles with relatively small effects of matrix interference, a textile sample may be directly inserted into the sample inlet 2 of the ion mobility spectrometer using a specifically designed clamper. Volatile SVHCs contained in the textile sample can be rapidly vaporized by the heating effect of a thermal desorber disposed at the inlet end and introduced into the interior of the instrument through the semipermeable membrane 3.

Herein, the design of said clamper 20 for sample introduction is exemplified as shown in Fig. 2. The clamper 20 comprises two sheet-like pieces 21 and 22 hinged together, and each of the two sheet-like pieces 21 and 22 has a window 23. When the two sheet-like pieces 21 and 22 are folded together, the windows 23 of the two sheet-like pieces 21 and 22 may be roughly overlapped. At least a part of the edges of the windows 23 of the two sheet-like pieces 21 and 22 may have a plurality of sieve pores 24. For example, the clamper may be an openable double-layer stacked structure and is formed by stacking an upper and a lower metal sheets 21 and 22 which are sheet-like pieces having exactly the same shape and size. The connected end is a rectangular feeding end and is provided with a rectangular window 23. A plurality of sieve pores 24 are provided at the edges on both sides. The textile sample 25 is prepared by cutting to exactly fit the size of the rectangular window. The other end of the clamper 20 may be opened.

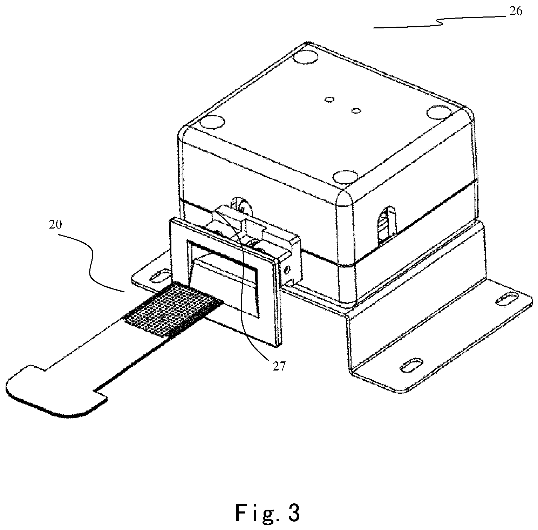

What is shown in Fig. 3 is a schematic diagram showing the structure of a sample introduction unit of an ion mobility spectrometer and how to perform direct sample introduction with a textile sample clamper. A textile sample 25 is placed at a position corresponding to the window 23 and inserted into a sample inlet 27 of a sample introduction 26 of the ion mobility spectrometer so as to ensure that the inserted position of the clamper exactly allows the textile sample exposed to the window is completely located above the heating area of the sample introduction unit 26 of the ion mobility spectrometer. In this way, SVHCs contained in the sample may be sufficiently and uniformly heated so as to be vaporized rapidly and introduced into the instrument for analysis. The materials used for the said metal sheet comprise stainless steel, copper, or aluminum.

For example, according to one embodiment of this invention, the method for rapidly detecting volatile SVHCs in a textile by means of directly inserting the textile sample to a sample inlet of the instrument comprises the steps of:
(1) cutting blank samples of a textile which is confirmed to be free of volatile SVHCs into pieces with a suitable size (a fixed size slightly smaller than the heating area of the sample inlet of the ion mobility spectrometer), accurately weighing them, and placing them in a specifically designed clamper to be clamped;
(2) depositing 1-10µl of standard solutions of volatile SVHCs (500-5000ppm) onto the textile samples clamped by the clamper respectively, standing them in the air for about 1min, and serving as a series of spiked samples of a textile containing volatile SVHCs after air drying of the organic solvent;
(3) directly inserting the spiked samples of the textile to the sample inlet of the instrument using the clamper, placing them for 6-10s and withdrawing, and measuring drift times and peak intensities of corresponding characteristic peaks in the ion mobility spectra, wherein the analysis time required for each sample is less than 30s;
(4) determining the threshold values corresponding to different detection limits according to the relationship between the peak intensities and the deposited amounts of SVHC standard solutions described above, selecting appropriate thresholds according to the masses of textile samples and the requirements for actual detection limits by the user (10ppm-1000ppm), and establishing a standard substance library of the SHVCs described above;
(5) cutting the textile sample to be tested into pieces with a suitable size following the same procedure as described in (1), accurately weighing the sample, clamping it with a specifically designed clamper, directly inserting it into the sample inlet of the instrument, and keeping it heated for 6-10s before removal; if a peak characteristic of a volatile SVHC is present in the ion mobility spectrum of the textile sample and the peak intensity exceeds or is equal to its corresponding threshold value, determining the textile sample to be positive and generating an alarm; and if in the ion mobility spectrum of the textile sample no peak characteristic of a volatile SVHC is observed or a peak characteristic of said volatile SVHC is present but the peak intensity of this peak is less than said threshold value, determining the textile sample to be negative; and estimating the concentration of the volatile SVHC in the textile sample based on the weight of the textile sample and the intensity of the peak characteristic of the volatile SVHC in the ion mobility spectrum.

According to one embodiment of this invention, in the process of determining the peak intensity thresholds of volatile SVHCs in textile samples and detecting target textile samples, it is preferred that said spiked textile samples have roughly the same size and weight as the textile samples to be tested.

Said textile samples comprise polyester cotton, terylene, cotton, cotton spandex, or the like. Said volatile SVHCs comprise 2-ethoxyethyl acetate, dibutyl phthalate, di-isobutyl phthalate,benzyl butyl phthalate, 1-methylpyrrolidone, 2,4-dinitrotoluene, anthracene, hydrazine hydrate, di-isodecyl phthalate, di-n-octyl phthalate, etc. On the other hand, as for SVHCs with relatively low volatility and textiles with relatively significant matrix interference, volatile SVHCs contained in the textile samples may be rapidly vaporized and introduced into the interior of the instrument through a semipermeable membrane 3, under the heating effect of a thermal desorber provided at the sample inlet end, by means of depositing an extract onto a sampling substrate and inserting the substrate into the sample inlet 2 of the instrument.

For example, according to another embodiment of this invention, the method for rapidly detecting volatile SVHCs in a textile by means of depositing an extract onto a sampling substrate and then inserting the substrate into the sample inlet 2 of the instrument comprises the steps of:
(1) accurately weighing 0.5g of textile blank sample which are confirmed to be free of any SVHCs described above, cutting them up, and placing them in 25ml test tubes with stoppers;
(2) adding 50-635µl of standard solutions of volatile SVHCs (1000ppm) thereon, and serving as a series of spiked samples of a textile containing volatile SVHCs after volatilization of the solvent;
(3) adding 10ml of an organic solvent, performing ultrasonic extraction for 5min, taking the supernatant 1-20µl and depositing on a sampling substrate, standing for 1-2min, inserting the substrate into a sample inlet of an ion mobility spectrometer after air drying of the organic solvent, and measuring the drift times and peak intensities of corresponding characteristic peaks in ion mobility spectra, wherein the analysis time required for each sample is less than 30s;
(4) determining thresholds corresponding to different detection limits according to the relationship between the peak intensities and the deposited amounts of the standard solutions of the volatile SVHCs, selecting appropriate thresholds according to the masses of textile samples and the requirements for actual detection limits by the user (80ppm-1000ppm), and establishing a standard substance library of the volatile SVHCs; and(5) accurately weighing 0.5 g of textile samples to be tested, adding 10ml of an organic solvent to the samples after being cut up, performing ultrasonic extraction for 5min, taking the supernatant 1-20µl and depositing on a sampling substrate, standing for 1min, and inserting the substrate into a sampling inlet of an ion mobility spectrometer after air drying of the organic solvent; if a peak characteristic of a volatile SVHC is present in the ion mobility spectrum of the textile sample and the peak intensity exceeds or is equal to its corresponding threshold value, determining the textile sample to be positive and generating an alarm; and if in the ion mobility spectrum of the textile sample no peak characteristic of a volatile SVHC is observed or a peak characteristic of said volatile SVHC is present but the peak intensity of this peak is less than said threshold value, determining the textile sample to be negative; and estimating the concentration of the volatile SVHC in the textile sample based on the weight of the textile sample and the intensity of the peak characteristic of the volatile SVHC in the ion mobility spectrum..

According to one embodiment of this invention, in the process of determining the peak intensity thresholds of volatile SVHCs in a textile and detecting a textile sample to be tested, said sampling substrates are completely the same, and the material thereof comprises a metal sheet or mesh, a sampling swab, or a polytetrafluoroethylene sheet. Said organic solvent for extraction comprises acetone, ethanol, chloroform, or methanol, which is capable of dissolving volatile SVHCs.

Said textile samples comprise polyester cotton, terylene, cotton, cotton spandex, wool fabric, or the like. Said volatile SVHCs comprise dibutyl phthalate, di-isobutyl phthalate, benzyl butyl phthalate, 1-methylpyrrolidone, 2,4-dinitrotoluene, anthracene, di-isodecyl phthalate, di-n-octyl phthalate, hexabromocyclododecane and diastereomers thereof, etc.

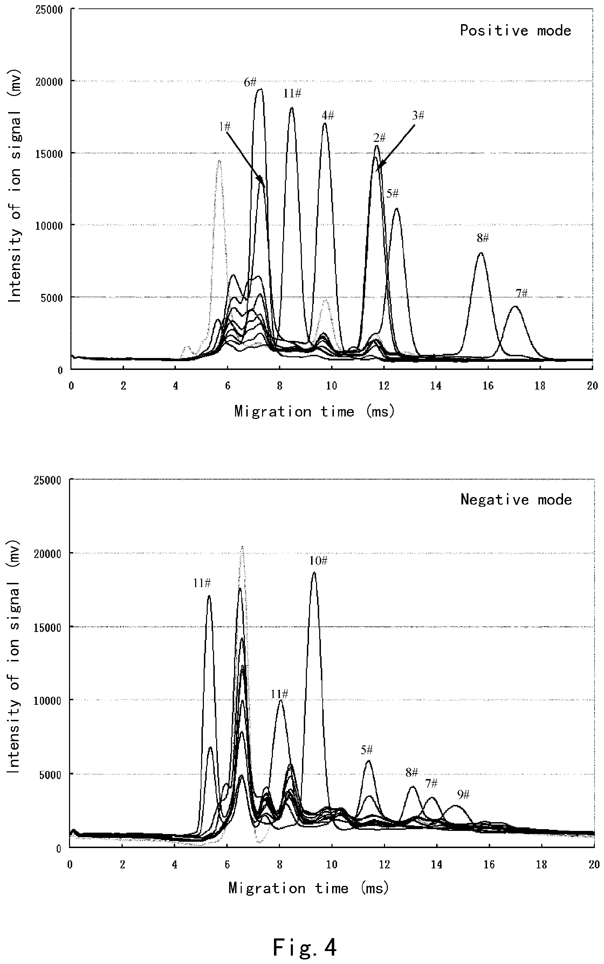

Figs. 4 to 9 provide a few experimental spectra obtained by using an ion mobility spectrometer to describe this invention. The instrument operates under the following conditions: using purified air as a carrier gas and a drift gas, setting the temperature of a positive-mode drift tube at 190°C, setting the temperature of a negative-mode migration tube at 130°C, and setting the temperature of a desorber at 190°C.

Fig. 4 shows ion mobility spectra obtained by detecting standard samples of 11 kinds of volatile SVHCs using an ion mobility spectrometer (100ppm, deposited amount of 1-10µl). The upper diagram corresponds to a positive-mode spectrum and the lower diagram corresponds to a negative-mode spectrum. It can be seen from the diagrams that all of 11 kinds of volatile SVHCs generate noticeable response signals, wherein some substances exhibit peaks in the positive mode, some substances exhibit peaks in the negative mode, and some substances exhibit peaks in both positive and negative modes, and the characteristic peak positions thereof are as shown in the following table.

| **Sample No.** | **Sample Name** | **Positive-mode peak position (ms)** | **Negative-mode peak position (ms)** |
|---|---|---|---|
| 1# | 2-ethoxyethyl acetate | 7.43 | |
| 2# | Dibutyl phthalate | 11.73 | |
| 3# | Di-isobutyl phthalate | 11.68 | |
| 4# | 1-methylpyrrolidone | 9.75 | |
| 5# | Butylbenzyl phthalate | 12.49 | 11.35 |
| 6# | Hydrazine hydrate | 7.41 | |
| 7# | Di-isodecyl phthalate | 17.04 | 13.81 |
| 8# | Di-n-octyl phthalate | 15.72 | 13.09 |
| 9# | Hexabromocyclododecane and diastereomers thereof | | 14.69 |
| 10# | 2,4-dinitrotoluene | | 9.31 |
| 11# | Anthracene | 8.48 | 5.38, 8.06 |

In Fig. 5, the two upper diagrams show positive and negative mode ion mobility spectra, obtained by a ion mobility spectrometer using the method provided in embodiment 1, wherein blank samples of cotton spandex cloth which are confirmed to be free of 11 volatile SVHCs are directly introduced using a specifically designed clamper; and the two lower diagrams show the superimposed ion mobility spectra detected in both positive and negative mode using the method provided in embodiment 1, obtained by means of direct introduction of spiked cotton spandex cloth samples on which 10 kinds of volatile SVHCs are deposited respectively using a specifically designed clamper. By comparing the two (upper and lower) positive-mode spectra and two (upper and lower) negative-mode spectra respectively, it can be seen that this method may sensitively detect 10 kinds of volatile SVHCs with concentrations no more than 140ppm by setting reasonable thresholds.

The two upper diagrams in Fig. 6 show positive and negative ion mobility spectra obtained by an ion mobility spectrometer using the method provided in embodiment 2, wherein simple solvent extraction was on blank samples of wool fabric which are confirmed to be free of 11 kinds of volatile SVHCs; and the two lower diagrams in Fig. 6 show the superimposed ion mobility spectra detected in both positive and negative mode using the method provided in embodiment 2, obtained by means of simple solvent extraction of spiked wool fabric samples on which 10 kinds of volatile SVHCs are deposited respectively.. By comparing the two (upper and lower) positive-mode spectra and two (upper and lower) negative-mode spectra respectively, it can be seen that this method may sensitively detect 9 kinds of volatile SVHCs having concentrations of 400ppm by setting reasonable thresholds.

It can be seen that 10 kinds and 9 kinds of said volatile SVHCs may be sensitively, rapidly, and effectively detected respectively by adopting the two embodiments. Herein, embodiment 1 is more convenient and may avoid the effects caused by the characteristic peaks of organic solvent, but the interference effects brought by direct introduction of textile samples are stronger, and it is suitable for substances which have relatively high volatility and characteristic peak positions close to those of the organic solvent, such as 2-ethoxyethyl acetate, hydrazine hydrate, etc.; while embodiment 2 requires a simple pretreatment step, but since the interference effect brought by matrices of textile samples is small, it is suitable for substances which have relatively low volatility and relatively low sensitivity when detected by means of direct sample introduction as described in embodiment 1, such as hexabromocyclododecane and diastereomers thereof, di-isodecyl phthalate, etc.

Advantages and positive effects of this invention are in that: A method for rapidly detecting volatile SVHCs in a textile using an ion mobility spectrometry technique is provided. Rapid detection and sound-and-light alarming of 11 kinds of volatile SVHCs may be achieved within a short time (1-6 minutes), respectively, by direct introduction of textile samples to be tested or combining an efficient and convenient solvent extraction pretreatment step. The detection limit of this method is less than 100ppm, which absolutely meets the requirement for the mass fraction limit of 1000ppm prescribed in EU REACH Regulation. The alarming thresholds may be determined according to the actual demands of users. This method may be used as a technique for rapid screening of these volatile SVHCs.

If necessary, in practical use, only samples which generate alarming signals need to be subject to accurate qualitative and quantitative analysis. Compared to conventional detection methods, due to the absence of external gas source and independent display, this instrument features small and good portability for facilitating field detection, rapid analysis and low requirements for sample pretreatment to greatly shorten the detection period and save the detection cost. It is particularly suitable for related examination agencies to perform simple and rapid detection and primary screening of a large batch of samples. However, due to limited performance on some aspect, it is not as good as the methods such as GC-MS, HPLC, etc, in terms of reproducibility and accurate quantification capability. In practical work, both kinds of methods may be combined to achieve rapid and accurate detection from primary screening to quantification, and meanwhile, it also provides the possibility to realize online detection of related substances in the production of textile industry.

## Claims

1. A method for detecting a volatile substance in a textile, comprising:
adopting an ion mobility spectrometer (1) as a detection means to analyze a textile sample (25) and obtain its ion mobility spectrum, either by means of directly inserting the textile sample to a sample inlet (2) of the spectrometer, or in combination with a solvent extraction pretreatment step, by depositing the obtained extract onto a sampling substrate and inserting the substrate into the sample inlet of the spectrometer;
if a peak characteristic of the volatile substance is present in the ion mobility spectrum of the textile sample and the peak intensity exceeds or is equal to a corresponding threshold value, determining the textile sample to be positive and generating an alarm; and
if in the ion mobility spectrum of the textile sample no peak characteristic of the volatile substance is observed or a peak characteristic of said volatile substance is present but the peak intensity of this peak is lower than said threshold value, determining the textile sample to be negative; and estimating the concentration of the volatile substance in the textile sample based on the weight of the textile sample and the intensity of the peak characteristic of the volatile substance in the ion mobility spectrum,
wherein said volatile substance belongs to a group of substances comprising: 2-ethoxyethyl acetate, dibutyl phthalate, di-isobutyl phthalate, butylbenzyl phthalate, 1-methylpyrrolidone, 2,4-dinitrotoluene, anthracene, hydrazine hydrate, di-isodecyl phthalate, and di-n-octyl phthalate.

2. The method for detecting a volatile substance in a textile according to claim 1, wherein when a textile sample of the textile is measured to obtain an ion mobility spectrum by means of direct introduction of the textile sample to a sample inlet of an ion mobility spectrometer, said method comprises the steps of:
step 1, cutting blank samples of a textile into pieces with a predetermined size, accurately weighing them, and placing them in a specifically designed sample clamper;
step 2, depositing standard solutions of the volatile substance with different concentrations thereon to prepare a series of spiked samples of a textile containing the volatile substance;
step 3, directly inserting the spiked samples to the sample inlet of the ion mobility spectrometer to obtain corresponding ion mobility spectra;
step 4, recording characteristic peak positions and peak intensities corresponding to said volatile substance in the ion mobility spectra, determining a detection threshold according to the relationship between the peak intensities and the deposited amounts of said volatile substance, and establishing a standard substance library of said volatile substance; and
step 5, cutting textile samples to be tested into pieces with a predetermined size, accurately weighing them, placing them in a specifically designed sample clamper, and inserting them in the sample inlet of the ion mobility spectrometer to obtain corresponding ion mobility spectra; if in the ion mobility spectrum of the textile sample a characteristic peak of the volatile substance is present and the peak intensity of this peak exceeds or is equal to the detection threshold set in the standard substance library, determining the textile sample to be positive and generating an alarming signal; and if in the ion mobility spectrum of the textile sample no characteristic peak of the volatile substance is present or a characterisic peak of said volatile substance is present but the peak intensity of this peak is less than the detection threshold set in the standard substance library, determining the textile sample to be negative; and the concentration of the substance in the textile sample may be estimated according to the weight of the textile sample and the peak intensity of the characteristic peak corresponding to the substance in the ion mobility spectrum.

3. The method for detecting a volatile substance in a textile according to claim 2, wherein in said step 1, the sizes and weights of respective spiked samples of said textile are roughly the same.

4. The method for detecting a volatile substance in a textile according to claim 2, wherein in said step 1, said clamper comprises two sheet-like pieces (21, 22) connected together, and each of the two sheet-like pieces has a window (23).

5. The method for detecting a volatile substance in a textile according to claim 4, wherein when said two sheet-like pieces are combined together, and the windows of the two sheet-like pieces are roughly overlapped.

6. The method for detecting a volatile substance in a textile according to claim 4 or 5, wherein at least a part of the edges of the windows of the two sheet-like pieces have a plurality of sieve pores (24).

7. The method for detecting a volatile substance in a textile according to claim 4 or 5, wherein said sheet-like piece is a metal sheet, the material of which comprises stainless steel, copper, or aluminum.

8. The method for detecting a volatile substance in a textile according to claim 2, wherein in said step 5, the sizes and weights of respective textile samples to be tested are roughly the same.

9. The method for detecting a volatile substance in a textile according to claim 8, wherein said textile samples to be tested have roughly the same sizes and weights as the respective spiked samples of said textile.

10. The method for detecting a volatile substance in a textile according to claim 2, wherein the types of said textile samples comprise at least one of polyester cotton, terylene, cotton, and cotton spandex.

11. The method for detecting a volatile substance in a textile according to claim 1, wherein when a textile sample is measured by means of depositing an extract onto a sampling substrate and inserting the substrate into the sample inlet of an ion mobility spectrometer in combination with a solvent extraction pretreatment step, said method comprises the steps of:
step 1, accurately weighing 0.5 g of blank samples of a textile, and cutting them up;
step 2, depositing standard solutions of the volatile substance with different concentrations thereon to prepare a series of spiked samples of a textile containing the volatile substance;
step 3, after evaporation of the solvent, adding 10ml of an organic solvent, performing ultrasonic extraction for 5min, taking the supernatant 1-20µl and depositing on a sampling substrate, standing for 1min, and inserting the substrate into the sample inlet of the ion mobility spectrometer after air drying of the organic solvent;
step 4, recording characteristic peak positions and peak intensities corresponding to said volatile substance in the ion mobility spectra, determining a detection threshold according to the relationship between the peak intensities and the deposited amounts of said volatile substance, and establishing a standard substance library of said volatile substance;
step 5, accurately weighing and cutting up 0.5 g of textile samples to be tested, adding 10ml of an organic solvent, performing ultrasonic extraction for 5min, taking the supernatant 1-20µl and depositing on a sampling substrate, standing for 1-2min, and inserting the substrate into the sample inlet of the ion mobility spectrometer after air drying of the organic solvent; and
step 6, if in the ion mobility spectrum of the textile sample a characteristic peak of the volatile substance is present and the peak intensity of this peak exceeds or is equal to the detection threshold set in the standard substance library, determining the textile sample to be positive and generating an alarming signal; and if in the ion mobility spectrum of the textile sample no characteristic peak of the volatile substance is present or a characteristic peak of said volatile substance is present but the peak intensity of this peak is lower than the detection threshold set in the standard substance library, determining the textile sample to be negative; and the concentration of the substance in the textile sample may be estimated according to the weight of the textile sample and the peak intensity of the characteristic peak corresponding to the substance in the ion mobility spectrum.

12. The method for detecting a volatile substance in a textile according to claim 11, wherein in said steps 3 and 5, said organic solvent for extraction comprises acetone, ethanol, chloroform, or methanol, which is capable of dissolving the volatile substance, or said sampling substrate comprises a metal sheet or mesh, a sampling swab, or a polytetrafluoroethylene sheet.

13. The method for detecting a volatile substance in a textile according to claim 11, wherein the types of said textile samples comprise at least one of polyester cotton, terylene, cotton, cotton spandex, and wool fabric.

## Patentansprüche

1. Verfahren zum Nachweisen eines flüchtigen Stoffs in einer Textilie, umfassend:
Verwenden eines Ionenmobilitätsspektrometers (1) als Nachweisvorrichtung zum Analysieren einer Textilprobe (25) und Erhalten ihres Ionenmobilitätsspektrums, entweder durch direktes Einführen der Textilprobe in einen Probeneinlass (2) des Spektrometers oder in Kombination mit einem Lösungsmittelextraktion-Vorbehandlungsschritt durch Aufbringen des erhaltenen Extrakts auf ein Probenahmesubstrat und Einführen des Substrats in den Probeneinlass des Spektrometers;
wenn ein Maximum-Merkmal des flüchtigen Stoffs in dem Ionenmobilitätsspektrum der Textilprobe vorhanden ist und die Maximum-Intensität einen entsprechenden Schwellenwert übersteigt oder ihm gleich ist, Einstufen der Textilprobe als positiv und Erzeugen eines Alarms; und
wenn in dem Ionenmobilitätsspektrum der Textilprobe kein Maximum-Merkmal des flüchtigen Stoffs beobachtet wird oder wenn ein Maximum-Merkmal des flüchtigen Stoffs vorhanden ist, aber die Maximum-Intensität dieses Maximums niedriger als der Schwellenwert ist, Einstufen der Textilprobe als negativ; und Abschätzen der Konzentration des flüchtigen Stoffs in der Textilprobe auf der Grundlage des Gewichts der Textilprobe und der Intensität des Maximum-Merkmals des flüchtigen Stoffs in dem Ionenmobilitätsspektrum,
wobei der flüchtige Stoff zu einer Gruppe von Stoffen gehört, die umfasst: 2-Ethoxyethylacetat, Dibutylphthalat, Diisobutylphthalat, Butylbenzylphthalat, 1-Methylpyrrolidon, 2,4-Dinitrotoluol, Anthracen, Hydrazinhydrat, Diisodecylphthalat und Di-n-octylphthalat.

2. Verfahren zum Nachweisen eines flüchtigen Stoffs in einer Textilie gemäß Anspruch 1, wobei, wenn eine Textilprobe der Textilie zum Erhalten eines Ionenmobilitätsspektrums durch direktes Einführen der Textilprobe in einen Probeneinlass eines Ionenmobilitätsspektrometers vermessen wird, das Verfahren die Schritte umfasst:
Schritt 1, Schneiden von Leerproben einer Textilie in Stücke mit einer vorbestimmten Größe, genaues Abwiegen davon und Anordnen in einer speziell gestalteten Probenklemmvorrichtung;
Schritt 2, Aufbringen von Standardlösungen des flüchtigen Stoffs mit verschiedenen Konzentrationen darauf, um eine Reihe von dotierten Proben einer Textilie, die den flüchtigen Stoff enthalten, herzustellen;
Schritt 3, direktes Einführen der dotierten Proben in den Probeneinlass des Ionenmobilitätsspektrometers, um entsprechende Ionenmobilitätsspektren zu erhalten;
Schritt 4, Aufzeichnen charakteristischer Maximum-Positionen und Maximum-Intensitäten, die dem flüchtigen Stoff entsprechen, in den Ionenmobilitätsspektren, Bestimmen einer Nachweisschwelle entsprechend der Beziehung zwischen den Maximum-Intensitäten und den aufgebrachten Mengen des flüchtigen Stoffs, und
Erstellen einer Standard-Stoffbibliothek für den flüchtigen Stoff; und
Schritt 5, Schneiden von zu prüfenden Textilproben in Stücke mit einer vorbestimmten Größe, genaues Abwiegen davon, Anordnen in einer speziell gestalteten Probenklemmvorrichtung und Einführen in den Probeneinlass des Ionenmobilitätsspektrometers, um entsprechende Ionenmobilitätsspektren zu erhalten; wenn in dem Ionenmobilitätsspektrum der Textilprobe ein charakteristisches Maximum des flüchtigen Stoffs vorhanden ist und die Maximum-Intensität dieses Maximums die in der Standard-Stoffbibliothek eingegebene Nachweisschwelle übersteigt oder ihr gleich ist, Einstufen der Textilprobe als positiv und Erzeugen eines Alarmsignals; und wenn in dem Ionenmobilitätsspektrum der Textilprobe kein charakteristisches Maximum des flüchtigen Stoffs vorhanden ist oder ein charakteristisches Maximum des flüchtigen Stoffs vorhanden ist aber die Maximum-Intensität dieses Maximums kleiner als die in der Standard-Stoffbibliothek eingegebene Nachweisschwelle ist, Einstufen der Textilprobe als negativ; wobei die Konzentration des Stoffs in der Textilprobe entsprechend dem Gewicht der Textilprobe und der Maximum-Intensität des charakteristischen Maximums, das dem Stoff entspricht, in dem Ionenmobilitätsspektrum abgeschätzt werden kann.

3. Verfahren zum Nachweisen eines flüchtigen Stoffs in einer Textilie gemäß Anspruch 2, wobei bei Schritt 1 die Größen und Gewichte von entsprechenden dotierten Proben der Textilie etwa gleich sind.

4. Verfahren zum Nachweisen eines flüchtigen Stoffs in einer Textilie gemäß Anspruch 2, wobei bei Schritt 1 die Klemmvorrichtung zwei plattenartige Stücke (21, 22) umfasst, die miteinander verbunden sind, und jedes der beiden plattenartigen Stücke ein Fenster (23) aufweist.

5. Verfahren zum Nachweisen eines flüchtigen Stoffs in einer Textilie gemäß Anspruch 4, wobei, wenn die beiden plattenartigen Stücke miteinander kombiniert werden, die die Fenster der beiden plattenartigen Stücke etwa überlappen.

6. Verfahren zum Nachweisen eines flüchtigen Stoffs in einer Textilie gemäß Anspruch 4 oder 5, wobei wenigstens ein Teil der Ränder der Fenster der beiden plattenartigen Stücke eine Vielzahl von Siebporen (24) aufweist.

7. Verfahren zum Nachweisen eines flüchtigen Stoffs in einer Textilie gemäß Anspruch 4 oder 5, wobei das plattenartige Stück ein Metallblech ist, dessen Material rostfreien Stahl, Kupfer oder Aluminium umfasst.

8. Verfahren zum Nachweisen eines flüchtigen Stoffs in einer Textilie gemäß Anspruch 2, wobei bei Schritt 5 die Größen und Gewichte von entsprechenden zu prüfenden Textilproben etwa gleich sind.

9. Verfahren zum Nachweisen eines flüchtigen Stoffs in einer Textilie gemäß Anspruch 8, wobei die zu prüfenden Textilproben etwa die gleichen Größen und Gewichte wie die entsprechenden dotierten Proben der Textilie aufweisen.

10. Verfahren zum Nachweisen eines flüchtigen Stoffs in einer Textilie gemäß Anspruch 2, wobei die Typen der Textilproben wenigstens eines von Polyester-Baumwolle, Terylen, Baumwolle und Baumwoll-Spandex umfassen.

11. Verfahren zum Nachweisen eines flüchtigen Stoffs in einer Textilie gemäß Anspruch 1, wobei, wenn die Textilprobe durch Aufbringen eines Extrakts auf ein Probenahmesubstrat und Einführen des Substrats in den Probeneinlass eines Ionenmobilitätsspektrometers in Kombination mit einem Lösungsmittelextraktion-Vorbehandlungsschritt vermessen wird, das Verfahren die Schritte umfasst:
Schritt 1, genaues Abwiegen von 0,5 g Leerproben einer Textilie und Zerschneiden davon;
Schritt 2, Aufbringen von Standardlösungen des flüchtigen Stoffs mit verschiedenen Konzentrationen darauf, um eine Reihe von dotierten Proben einer Textilie, die den flüchtigen Stoff enthalten, herzustellen;
Schritt 3, nach Abdampfen des Lösungsmittels Zugeben von 10 ml eines organischen Lösungsmittels, Durchführen von 5 min Ultraschallextraktion, Entnehmen von 1-20 µl des Überstands und Aufbringen auf ein Probenahmesubstrat, 1 min Stehenlassen und nach Lufttrocknen des organischen Lösungsmittels Einführen des Substrats in den Probeneinlass des Ionenmobilitätsspektrometers;
Schritt 4, Aufzeichnen charakteristischer Maximum-Positionen und Maximum-Intensitäten, die dem flüchtigen Stoff entsprechen, in den Ionenmobilitätsspektren, Bestimmen einer Nachweisschwelle entsprechend der Beziehung zwischen den Maximum-Intensitäten und den aufgebrachten Mengen des flüchtigen Stoffs, und Erstellen einer Standard-Stoffbibliothek für den flüchtigen Stoff;
Schritt 5, genaues Abwiegen und Zerschneiden von 0,5 g von zu prüfenden Textilproben, Zugeben von 10 ml eines organischen Lösungsmittels, Durchführen von 5 min Ultraschallextraktion, Entnehmen von 1-20 µl des Überstands und Aufbringen auf ein Probenahmesubstrat, 1-2 min Stehenlassen und nach Lufttrocknen des organischen Lösungsmittels Einführen des Substrats in den Probeneinlass des Ionenmobilitätsspektrometers; und
Schritt 6, wenn in dem Ionenmobilitätsspektrum der Textilprobe ein charakteristisches Maximum des flüchtigen Stoffs vorhanden ist und die Maximum-Intensität dieses Maximums die in der Standard-Stoffbibliothek eingegebene Nachweisschwelle übersteigt oder ihr gleich ist, Einstufen der Textilprobe als positiv und Erzeugen eines Alarmsignals; und wenn in dem Ionenmobilitätsspektrum der Textilprobe kein charakteristisches Maximum des flüchtigen Stoffs vorhanden ist oder ein charakteristisches Maximum des flüchtigen Stoffs vorhanden ist aber die Maximum-Intensität dieses Maximums kleiner als die in der Standard-Stoffbibliothek eingegebene Nachweisschwelle ist, Einstufen der Textilprobe als negativ; wobei die Konzentration des Stoffs in der Textilprobe entsprechend dem Gewicht der Textilprobe und der Maximum-Intensität des charakteristischen Maximums, das dem Stoff entspricht, in dem Ionenmobilitätsspektrum abgeschätzt werden kann.

12. Verfahren zum Nachweisen eines flüchtigen Stoffs in einer Textilie gemäß Anspruch 11, wobei bei den Schritten 3 und 5 das organische Lösungsmittel für die Extraktion Aceton, Ethanol, Chloroform oder Methanol umfasst, das fähig ist, den flüchtigen Stoff zu lösen, oder das Probenahmesubstrat ein Metallblech oder -netz, einen Probenahmetupfer oder ein Polytetrafluorethylenblatt umfasst.

13. Verfahren zum Nachweisen eines flüchtigen Stoffs in einer Textilie gemäß Anspruch 11, wobei die Typen der Textilproben wenigstens eines von Polyester-Baumwolle, Terylen, Baumwolle, Baumwoll-Spandex und Wollgewebe umfassen.

## Revendications

1. Procédé de détection d'une substance volatile dans un textile, comprenant :
l'adoption d'un spectromètre de mobilité ionique (1) en tant que moyen de détection pour analyser un échantillon de textile (25) et obtenir son spectre de mobilité ionique, au moyen de l'insertion de l'échantillon de textile directement dans une entrée d'échantillon (2) du spectromètre, ou en combinaison avec une étape de prétraitement d'extraction par solvant, par dépôt de l'extrait obtenu sur un substrat d'échantillonnage et insertion du substrat dans l'entrée d'échantillon du spectromètre ;
si un pic caractéristique de la substance volatile est présent dans le spectre de mobilité ionique de l'échantillon de textile et l'intensité de pic dépasse ou est égale à une valeur de seuil correspondante, la détermination de l'échantillon de textile comme étant positif et la génération d'une alarme ; et
si, dans le spectre de mobilité ionique de l'échantillon de textile, aucun pic caractéristique de la substance volatile n'est observé ou un pic caractéristique de ladite substance volatile est présent mais l'intensité de pic de ce pic est inférieure à ladite valeur de seuil, la détermination de l'échantillon de textile comme étant négatif ; et l'estimation de la concentration de la substance volatile dans l'échantillon de textile sur la base du poids de l'échantillon de textile et de l'intensité du pic caractéristique de la substance volatile dans le spectre de mobilité ionique,
dans lequel ladite substance volatile appartient à un groupe de substances comprenant : acétate de 2-éthoxy-éthyle, phtalate de dibutyle, phtalate de diisobutyle, phtalate de butylbenzyle, 1-méthylpyrrolidone, 2,4-dinitrotoluène, anthracène, hydrate d'hydrazine, phtalate de diisodécyle et phtalate de di-n-octyle.

2. Procédé de détection d'une substance volatile dans un textile selon la revendication 1, dans lequel, lorsqu'un échantillon de textile du textile est mesuré pour obtenir un spectre de mobilité ionique au moyen de l'introduction directe de l'échantillon de textile dans une entrée d'échantillon d'un spectromètre de mobilité ionique, ledit procédé comprend les étapes de :
étape 1, découpe d'échantillons témoins d'un textile en pièces ayant une taille prédéterminée, pesage précis de ceux-ci, et placement de ceux-ci dans un dispositif de serrage d'échantillon spécifiquement conçu ;
étape 2, dépôt de solutions standard de la substance volatile ayant différentes concentrations sur ceux-ci pour préparer une série d'échantillons enrichis d'un textile contenant la substance volatile ;
étape 3, insertion des échantillons enrichis directement dans l'entrée d'échantillon du spectromètre de mobilité ionique pour obtenir des spectres de mobilité ionique correspondants ;
étape 4, enregistrement de positions et d'intensités de pic de pics caractéristiques correspondant à ladite substance volatile dans les spectres de mobilité ionique, détermination d'un seuil de détection selon la relation entre les intensités de pic et les quantités déposées de ladite substance volatile, et établissement d'une banque de substances standard de ladite substance volatile ; et
étape 5, découpe d'échantillons de textile d'essai en pièces ayant une taille prédéterminée, pesage précis de ceux-ci, placement de ceux-ci dans un dispositif de serrage d'échantillon spécifiquement conçu, et insertion de ceux-ci dans l'entrée d'échantillon du spectromètre de mobilité ionique pour obtenir des spectres de mobilité ionique correspondants ; si, dans le spectre de mobilité ionique de l'échantillon de textile, un pic caractéristique de la substance volatile est présent et l'intensité de pic de ce pic dépasse ou est égale au seuil de détection défini dans la banque de substances standard, détermination de l'échantillon de textile comme étant positif et génération d'un signal d'alarme ; et si,
dans le spectre de mobilité ionique de l'échantillon de textile, aucun pic caractéristique de la substance volatile n'est présent ou un pic caractéristique de ladite substance volatile est présent mais l'intensité de pic de ce pic est inférieure au seuil de détection défini dans la banque de substances standard, détermination de l'échantillon de textile comme étant négatif ; et la concentration de la substance dans l'échantillon de textile peut être estimée en fonction du poids de l'échantillon de textile et de l'intensité de pic du pic caractéristique correspondant à la substance dans le spectre de mobilité ionique.

3. Procédé de détection d'une substance volatile dans un textile selon la revendication 2, dans lequel, dans ladite étape 1, les tailles et poids d'échantillons enrichis respectifs dudit textile sont approximativement identiques.

4. Procédé de détection d'une substance volatile dans un textile selon la revendication 2, dans lequel, dans ladite étape 1, ledit dispositif de serrage comprend deux éléments en forme de feuille (21, 22) raccordés conjointement, et chacun des deux éléments en forme de feuille comporte une fenêtre (23).

5. Procédé de détection d'une substance volatile dans un textile selon la revendication 4, dans lequel, lorsque lesdits deux éléments en forme de feuille sont combinés conjointement, et les fenêtres des deux éléments en forme de feuille se chevauchent approximativement.

6. Procédé de détection d'une substance volatile dans un textile selon la revendication 4 ou 5, dans lequel au moins une partie des bords des fenêtres des deux éléments en forme de feuille comporte une pluralité de pores de tamis (24).

7. Procédé de détection d'une substance volatile dans un textile selon la revendication 4 ou 5, dans lequel ledit élément en forme de feuille est une feuille métallique, dont le matériau comprend de l'acier inoxydable, du cuivre ou de l'aluminium.

8. Procédé de détection d'une substance volatile dans un textile selon la revendication 2, dans lequel, dans ladite étape 5, les tailles et poids d'échantillons de textile d'essai respectifs sont approximativement identiques.

9. Procédé de détection d'une substance volatile dans un textile selon la revendication 8, dans lequel lesdits échantillons de textile d'essai ont approximativement les mêmes tailles et poids que les échantillons enrichis respectifs dudit textile.

10. Procédé de détection d'une substance volatile dans un textile selon la revendication 2, dans lequel les types desdits échantillons de textile comprennent au moins l'un parmi le coton de polyester, le térylène, le coton et le coton spandex.

11. Procédé de détection d'une substance volatile dans un textile selon la revendication 1, dans lequel, lorsqu'un échantillon de textile est mesuré au moyen du dépôt d'un extrait sur un substrat d'échantillonnage et de l'insertion du substrat dans l'entrée d'échantillon d'un spectromètre de mobilité ionique en combinaison avec une étape de prétraitement d'extraction par solvant, ledit procédé comprend les étapes de :
étape 1, pesage précis de 0,5 g d'échantillons témoins d'un textile, et découpe de ceux-ci ;
étape 2, dépôt de solutions standard de la substance volatile ayant différentes concentrations sur ceux-ci pour préparer une série d'échantillons enrichis d'un textile contenant la substance volatile ;
étape 3, après évaporation du solvant, ajout de 10 ml d'un solvant organique, conduite d'une extraction ultrasonore pendant 5 min, prélèvement de 1 à 20 µl de surnageant et dépôt sur un substrat d'échantillonnage, repos pendant 1 min, et insertion du substrat dans l'entrée d'échantillon du spectromètre de mobilité ionique après séchage par l'air du solvant organique ;
étape 4, enregistrement de positions et d'intensités de pic de pics caractéristiques correspondant à ladite substance volatile dans les spectres de mobilité ionique, détermination d'un seuil de détection selon la relation entre les intensités de pic et les quantités déposées de ladite substance volatile, et établissement d'une banque de substances standard de ladite substance volatile ;
étape 5, pesage précis et découpe de 0,5 g d'échantillons de textile d'essai, ajout de 10 ml d'un solvant organique, conduite d'une extraction ultrasonore pendant 5 min, prélèvement de 1 à 20 µl de surnageant et dépôt sur un substrat d'échantillonnage, repos pendant 1 à 2 min, et insertion du substrat dans l'entrée d'échantillon du spectromètre de mobilité ionique après séchage par l'air du solvant organique ; et
étape 6, si, dans le spectre de mobilité ionique de l'échantillon de textile, un pic caractéristique de la substance volatile est présent et l'intensité de pic de ce pic dépasse ou est égale au seuil de détection défini dans la banque de substances standard, détermination de l'échantillon de textile comme étant positif et génération d'un signal d'alarme ; et si, dans le spectre de mobilité ionique de l'échantillon de textile, aucun pic caractéristique de la substance volatile n'est présent ou un pic caractéristique de ladite substance volatile est présent mais l'intensité de pic de ce pic est inférieure au seuil de détection défini dans la banque de substances standard, détermination de l'échantillon de textile comme étant négatif ; et la concentration de la substance dans l'échantillon de textile peut être estimée en fonction du poids de l'échantillon de textile et de l'intensité de pic du pic caractéristique correspondant à la substance dans le spectre de mobilité ionique.

12. Procédé de détection d'une substance volatile dans un textile selon la revendication 11, dans lequel, dans lesdites étapes 3 et 5, ledit solvant organique pour l'extraction comprend l'acétone, l'éthanol, le chloroforme ou le méthanol, qui est capable de dissoudre la substance volatile, ou ledit substrat d'échantillonnage comprend une feuille ou une grille métallique, un écouvillon d'échantillonnage ou une feuille de polytétrafluoroéthylène.

13. Procédé de détection d'une substance volatile dans un textile selon la revendication 11, dans lequel les types desdits échantillons de textile comprennent au moins l'un parmi le coton de polyester, le térylène, le coton, le coton spandex et un tissu de laine.
